# EUROPÄISCHE PATENTANMELDUNG

(11) **EP 2 055 791 A1**
(43) Veröffentlichungstag der Anmeldung: **06.05.2009**
(21) Anmeldenummer: 08017732.2
(22) Anmeldetag: 09.10.2008
(51) Int. Cl.: C12Q 1/68

(54) **Verfahren zur parallelen Amplifikation von wenigstens zwei verschiedenen Nukleinsäureabschnitten**

(30) Priorität: 29.10.2007 DE 102007051578
(71) Anmelder: Olympus Life Science Research Europa GmbH, 81377 München (DE)
(72) Erfinder: Mann, Wolfgang, Dr., 95512 Neudrossenfeld (DE); Kirchner, Roland, Dr., 83533 Edling (DE)
(74) Vertreter: Manitz, Finsterwald & Partner GbR

(57) **Zusammenfassung**

Ein Verfahren zur Amplifikation von wenigstens zwei verschiedenen Nukleinsäureabschnitten aus einer Nukleinsäure enthaltenden Probe umfasst die nachfolgenden Schritte:
a) Bereitstellen einer Nukleinsäure enthaltenden Probe,
b) Zugabe von wenigstens zwei Primerpaaren zu der Probe, wobei jedes der wenigstens zwei Primerpaare für einen der wenigstens zwei verschiedenen, zu amplifizierenden Nukleinsäureabschnitte spezifisch ist,
c) Durchführen einer PCR-Reaktion mit der in dem Schritt b) erhaltenen Probe,
d) Aufteilen der in dem Schritt c) erhaltenen Probe in wenigstens zwei Teilmengen und Zugabe von jeweils wenigstens einem Primerpaar zu jeder der Teilmengen, wobei jedes den Teilmengen zugesetzte Primerpaar mit jeweils einem der in dem Schritt b) zugegebenen Primerpaare identisch ist und sich das/die den einzelnen Teilmengen zugegebene(n) Primerpaar(e) jeweils von dem/den den anderen Teilmengen zugegebenen Primerpaar(en) unterscheidet/unterscheiden, sowie
e) Durchführen einer PCR-Reaktion mit den in Schritt d) erhaltenen Teilmengen.

SEQUENCE LISTING

<110>  Advalytix AG

<120>  Verfahren zur parallelen Amplifikation von wenigstens zwei verschiedenen
Nukleinsäureabschnitten

<130>  A10294PEP

<160>  240

<170>  PatentIn version 3.4

<210>  1
<211>  19
<212>  DNA
<213>  Homo sapiens

<400>  1
tgccctcaca atcaggtgt                                                    19

<210>  2
<211>  18
<212>  DNA
<213>  Homo sapiens

<400>  2
gaaaaccacc cacgttgc                                                     18

<210>  3
<211>  20
<212>  DNA
<213>  Homo sapiens

<400>  3
tcacaaaagt gcggtgagac                                                   20

<210>  4
<211>  19
<212>  DNA
<213>  Homo sapiens

<400>  4
caaagctggg gctcctaag                                                    19

<210>  5
<211>  21
<212>  DNA
<213>  Homo sapiens

<400>  5
ctccatttgc tggataattc g                                                 21

<210>  6
<211>  20
<212>  DNA
<213>  Homo sapiens

<400>  6
actcttctgt ccacctggga                                                   20

<210>  7

<211>  18
<212>  DNA
<213>  Homo sapiens

<400>  7
ccgaacgctt tgaggcta                                                    18


<210>  8
<211>  19
<212>  DNA
<213>  Homo sapiens

<400>  8
gaggggattc gtgataccg                                                   19


<210>  9
<211>  23
<212>  DNA
<213>  Homo sapiens

<400>  9
actcagggat ttggagagaa ctt                                              23


<210>  10
<211>  19
<212>  DNA
<213>  Homo sapiens

<400>  10
agtgaccacc ccttggaac                                                   19


<210>  11
<211>  22
<212>  DNA
<213>  Homo sapiens

<400>  11
ggctattgca ggaaaatacg tc                                               22


<210>  12
<211>  18
<212>  DNA
<213>  Homo sapiens

<400>  12
ttcgcaaggt tactgggc                                                    18


<210>  13
<211>  20
<212>  DNA
<213>  Homo sapiens

<400>  13
agctttggga ggatgttcaa                                                  20


<210>  14
<211>  21
<212>  DNA
<213>  Homo sapiens

<400>  14

gccctaaaca aacggtgact t                                                21

<210>  15
<211>  21
<212>  DNA
<213>  Homo sapiens

<400>  15
tgtggcaatt taggcagagt c                                                21

<210>  16
<211>  18
<212>  DNA
<213>  Homo sapiens

<400>  16
ggggagaagc cctacgag                                                    18

<210>  17
<211>  19
<212>  DNA
<213>  Homo sapiens

<400>  17
aactgcgaag ggctcagat                                                   19

<210>  18
<211>  18
<212>  DNA
<213>  Homo sapiens

<400>  18
ctaatggtgc agggtcgc                                                    18

<210>  19
<211>  20
<212>  DNA
<213>  Homo sapiens

<400>  19
gtgccccgag agtgtttcta                                                  20

<210>  20
<211>  19
<212>  DNA
<213>  Homo sapiens

<400>  20
agacttcacg ctggccttt                                                   19

<210>  21
<211>  20
<212>  DNA
<213>  Homo sapiens

<400>  21
gaggcagagg ttctcactgg                                                  20

<210>  22
<211>  22

<212>  DNA
<213>  Homo sapiens

<400>  22
aacagacttt caacaatggc ct                                                22

<210>  23
<211>  20
<212>  DNA
<213>  Homo sapiens

<400>  23
ggtaatggag ccggtttaca                                                   20

<210>  24
<211>  18
<212>  DNA
<213>  Homo sapiens

<400>  24
aacacgcttt tggtccga                                                     18

<210>  25
<211>  19
<212>  DNA
<213>  Homo sapiens

<400>  25
tgaaagttga attgccgga                                                    19

<210>  26
<211>  23
<212>  DNA
<213>  Homo sapiens

<400>  26
tctgaatagt gcctctggga tac                                               23

<210>  27
<211>  21
<212>  DNA
<213>  Homo sapiens

<400>  27
cccagaaccc tgttattacc c                                                 21

<210>  28
<211>  20
<212>  DNA
<213>  Homo sapiens

<400>  28
tctgcgcttg taagacatcg                                                   20

<210>  29
<211>  18
<212>  DNA
<213>  Homo sapiens

<400>  29
gcaaaggcta ccgggatt                                                     18

<210> 30
<211> 23
<212> DNA
<213> Homo sapiens

<400> 30
aaaacacaaa ttcacctcct gtg 23

<210> 31
<211> 20
<212> DNA
<213> Homo sapiens

<400> 31
ttcaccccat caatttaggc 20

<210> 32
<211> 23
<212> DNA
<213> Homo sapiens

<400> 32
tctcatgtct aatacccacc agg 23

<210> 33
<211> 23
<212> DNA
<213> Homo sapiens

<400> 33
tgacttttga gttccttcag agc 23

<210> 34
<211> 19
<212> DNA
<213> Homo sapiens

<400> 34
gctgggtgag aatggttca 19

<210> 35
<211> 20
<212> DNA
<213> Homo sapiens

<400> 35
gcacccctgt aaccaagtgt 20

<210> 36
<211> 18
<212> DNA
<213> Homo sapiens

<400> 36
gccatcagtt gcggaatc 18

<210> 37
<211> 23
<212> DNA

<213>  Homo sapiens

<400>  37
ctgtgctgga aagtgtgact aca                                              23


<210>  38
<211>  23
<212>  DNA
<213>  Homo sapiens

<400>  38
accctgagtt gacagaatcg tag                                              23


<210>  39
<211>  23
<212>  DNA
<213>  Homo sapiens

<400>  39
agagagaata gccaaggtgt cct                                              23


<210>  40
<211>  19
<212>  DNA
<213>  Homo sapiens

<400>  40
tgtgtacgtc ctgatgcca                                                   19


<210>  41
<211>  18
<212>  DNA
<213>  Homo sapiens

<400>  41
ccaggacttt ccacacgg                                                    18


<210>  42
<211>  22
<212>  DNA
<213>  Homo sapiens

<400>  42
aagatgaccc cattctcaca gt                                               22


<210>  43
<211>  22
<212>  DNA
<213>  Homo sapiens

<400>  43
tgttgaagag tgcatcgagt tc                                               22


<210>  44
<211>  21
<212>  DNA
<213>  Homo sapiens

<400>  44
cagcaacgga cattctgagt t                                                21

<210> 45
<211> 21
<212> DNA
<213> Homo sapiens

<400> 45
cctggtctcc tgacaattac g 21


<210> 46
<211> 25
<212> DNA
<213> Homo sapiens

<400> 46
tgttttgtac tttgggtctc ctaga 25


<210> 47
<211> 22
<212> DNA
<213> Homo sapiens

<400> 47
agacccttca ttgttgtcca ag 22


<210> 48
<211> 21
<212> DNA
<213> Homo sapiens

<400> 48
tttgaggacg tttgatgttc c 21


<210> 49
<211> 23
<212> DNA
<213> Homo sapiens

<400> 49
tttgcctaaa agttacaagg ctg 23


<210> 50
<211> 21
<212> DNA
<213> Homo sapiens

<400> 50
attctgtgat aagcccgtga a 21


<210> 51
<211> 19
<212> DNA
<213> Homo sapiens

<400> 51
gccatgtacc tcagtgcgt 19


<210> 52
<211> 18
<212> DNA
<213> Homo sapiens

<400>  52
taagtccccg agcgtcag                                                    18

<210>  53
<211>  22
<212>  DNA
<213>  Homo sapiens

<400>  53
gccatatttt cacaggagca at                                               22

<210>  54
<211>  20
<212>  DNA
<213>  Homo sapiens

<400>  54
aacaaggttg gattatgccg                                                  20

<210>  55
<211>  20
<212>  DNA
<213>  Homo sapiens

<400>  55
ttcctatgtg ctagggtgcc                                                  20

<210>  56
<211>  23
<212>  DNA
<213>  Homo sapiens

<400>  56
aattgagttt gggagaaacc act                                              23

<210>  57
<211>  21
<212>  DNA
<213>  Homo sapiens

<400>  57
gaatcggaat taaccacgga t                                                21

<210>  58
<211>  22
<212>  DNA
<213>  Homo sapiens

<400>  58
aatgtaagtc atccccaggc ta                                               22

<210>  59
<211>  25
<212>  DNA
<213>  Homo sapiens

<400>  59
ctatcccgac cacttaggag ataat                                            25

<210>  60
<211>  19
<212>  DNA
<213>  Homo sapiens

<400>  60
aaggcacaac gtgaagcag                                                    19


<210>  61
<211>  20
<212>  DNA
<213>  Homo sapiens

<400>  61
ggtgtagtcc aatccgctgt                                                   20


<210>  62
<211>  20
<212>  DNA
<213>  Homo sapiens

<400>  62
aaagcatttc tcgatgtgcc                                                   20


<210>  63
<211>  18
<212>  DNA
<213>  Homo sapiens

<400>  63
gtgggcactc ctggtgac                                                     18


<210>  64
<211>  18
<212>  DNA
<213>  Homo sapiens

<400>  64
tgcgctgctg ttctcaaa                                                     18


<210>  65
<211>  19
<212>  DNA
<213>  Homo sapiens

<400>  65
taagccatcc aacactggc                                                    19


<210>  66
<211>  21
<212>  DNA
<213>  Homo sapiens

<400>  66
tcagccttca acaaatcgtc t                                                 21


<210>  67
<211>  23
<212>  DNA
<213>  Homo sapiens

<400>  67
tggctgtatc cctaatttca cag                                              23


<210>  68
<211>  18
<212>  DNA
<213>  Homo sapiens

<400>  68
gctgctgacc cattcagg                                                    18


<210>  69
<211>  25
<212>  DNA
<213>  Homo sapiens

<400>  69
agtgagttac actacccaca aggtc                                            25


<210>  70
<211>  19
<212>  DNA
<213>  Homo sapiens

<400>  70
tggaagccgg ttaagttcc                                                   19


<210>  71
<211>  19
<212>  DNA
<213>  Homo sapiens

<400>  71
gcaacgggga tggtacttt                                                   19


<210>  72
<211>  22
<212>  DNA
<213>  Homo sapiens

<400>  72
ctacacaata ggaaaggtcg cc                                               22


<210>  73
<211>  18
<212>  DNA
<213>  Homo sapiens

<400>  73
acaattccgt gggacgaa                                                    18


<210>  74
<211>  19
<212>  DNA
<213>  Homo sapiens

<400>  74
ggtcagaagt gccccaagt                                                   19


<210>  75

<211>  18
<212>  DNA
<213>  Homo sapiens

<400>  75
ggacacatag gctcccga                                                    18


<210>  76
<211>  20
<212>  DNA
<213>  Homo sapiens

<400>  76
tgcaaacatc ctcgcttaga                                                  20


<210>  77
<211>  19
<212>  DNA
<213>  Homo sapiens

<400>  77
gaatgctgtg gagacaccg                                                   19


<210>  78
<211>  19
<212>  DNA
<213>  Homo sapiens

<400>  78
gggagtgagg cccagatta                                                   19


<210>  79
<211>  20
<212>  DNA
<213>  Homo sapiens

<400>  79
ttggttaatc ttcgagccgt                                                  20


<210>  80
<211>  20
<212>  DNA
<213>  Homo sapiens

<400>  80
tccagtaaca ccccgagagt                                                  20


<210>  81
<211>  18
<212>  DNA
<213>  Homo sapiens

<400>  81
gcaaacagca cctcctgg                                                    18


<210>  82
<211>  21
<212>  DNA
<213>  Homo sapiens

<400>  82

tgacttgatt tgggaacctt g                                                21

<210>  83
<211>  23
<212>  DNA
<213>  Homo sapiens

<400>  83
ctgtgccata actctttacg gtc                                              23

<210>  84
<211>  18
<212>  DNA
<213>  Homo sapiens

<400>  84
catgggcctc tcagcatt                                                    18

<210>  85
<211>  23
<212>  DNA
<213>  Homo sapiens

<400>  85
tgaagcctag ataacctcag tgc                                              23

<210>  86
<211>  18
<212>  DNA
<213>  Homo sapiens

<400>  86
ctccttcgca acattcgg                                                    18

<210>  87
<211>  19
<212>  DNA
<213>  Homo sapiens

<400>  87
aatggatacc ctctggcga                                                   19

<210>  88
<211>  24
<212>  DNA
<213>  Homo sapiens

<400>  88
gtttgagttc tacgatcact tgga                                             24

<210>  89
<211>  18
<212>  DNA
<213>  Homo sapiens

<400>  89
ccctccattc ctgtgtgg                                                    18

<210>  90
<211>  21

<212>  DNA
<213>  Homo sapiens

<400>  90
agctccaggg tgagaaattg t                                                21


<210>  91
<211>  18
<212>  DNA
<213>  Homo sapiens

<400>  91
gggcttgggc tgtaatcc                                                    18


<210>  92
<211>  19
<212>  DNA
<213>  Homo sapiens

<400>  92
gtccgagggc atcctttag                                                   19


<210>  93
<211>  18
<212>  DNA
<213>  Homo sapiens

<400>  93
cttacccagc catcacgg                                                    18


<210>  94
<211>  24
<212>  DNA
<213>  Homo sapiens

<400>  94
agcattgaga caggtgtttg taac                                             24


<210>  95
<211>  21
<212>  DNA
<213>  Homo sapiens

<400>  95
gacagcaagc atatttgagg g                                                21


<210>  96
<211>  20
<212>  DNA
<213>  Homo sapiens

<400>  96
tctctgtgtc ataacgggca                                                  20


<210>  97
<211>  18
<212>  DNA
<213>  Homo sapiens

<400>  97
tcccaactaa tcccgcag                                                    18

<210> 98
<211> 19
<212> DNA
<213> Homo sapiens

<400> 98
ggggagaacc cacttctga 19

<210> 99
<211> 22
<212> DNA
<213> Homo sapiens

<400> 99
tctgcctgtg aatcttagtc ca 22

<210> 100
<211> 18
<212> DNA
<213> Homo sapiens

<400> 100
ttgtgctgcg gtcttgaa 18

<210> 101
<211> 18
<212> DNA
<213> Homo sapiens

<400> 101
tagcagccct ggtggaac 18

<210> 102
<211> 22
<212> DNA
<213> Homo sapiens

<400> 102
ttctctcctc ctagtatgcc ga 22

<210> 103
<211> 26
<212> DNA
<213> Homo sapiens

<400> 103
aaataactct gaaaggtgct cagttt 26

<210> 104
<211> 20
<212> DNA
<213> Homo sapiens

<400> 104
ctgaaatacg ctcgtggtca 20

<210> 105
<211> 18
<212> DNA

<213>  Homo sapiens

<400>  105
gcaccaggga gtctgcac                                                    18


<210>  106
<211>  22
<212>  DNA
<213>  Homo sapiens

<400>  106
ctagagttgc tgtttgggac ct                                               22


<210>  107
<211>  19
<212>  DNA
<213>  Homo sapiens

<400>  107
cactatgcgg tatgtcgcc                                                   19


<210>  108
<211>  18
<212>  DNA
<213>  Homo sapiens

<400>  108
tccttcttgg ggcagatg                                                    18


<210>  109
<211>  22
<212>  DNA
<213>  Homo sapiens

<400>  109
cttgccacaa accaagtgtt ta                                               22


<210>  110
<211>  18
<212>  DNA
<213>  Homo sapiens

<400>  110
ggagggtgtt aggcccat                                                    18


<210>  111
<211>  23
<212>  DNA
<213>  Homo sapiens

<400>  111
agtattggct tttctcaaga ggc                                              23


<210>  112
<211>  19
<212>  DNA
<213>  Homo sapiens

<400>  112
tccagagcta tcagtggcg                                                   19

<210>  113
<211>  19
<212>  DNA
<213>  Homo sapiens

<400>  113
cctccttccg gtgaaagtc                                                    19


<210>  114
<211>  18
<212>  DNA
<213>  Homo sapiens

<400>  114
ccgtgcctct atgaacgc                                                     18


<210>  115
<211>  20
<212>  DNA
<213>  Homo sapiens

<400>  115
tggcaagacc tggagaagtt                                                   20


<210>  116
<211>  21
<212>  DNA
<213>  Homo sapiens

<400>  116
caaagcccta aatgatgtcc a                                                 21


<210>  117
<211>  18
<212>  DNA
<213>  Homo sapiens

<400>  117
cggtgagaaa acgatggg                                                     18


<210>  118
<211>  19
<212>  DNA
<213>  Homo sapiens

<400>  118
aagaaggcgg tcagatgct                                                    19


<210>  119
<211>  18
<212>  DNA
<213>  Homo sapiens

<400>  119
tgtggcttct caatcccc                                                     18


<210>  120
<211>  19
<212>  DNA
<213>  Homo sapiens

<400>  120
ggctctaagt ggcttccca                                                    19


<210>  121
<211>  20
<212>  DNA
<213>  Homo sapiens

<400>  121
caggagtcgt tcagaggtcc                                                   20


<210>  122
<211>  23
<212>  DNA
<213>  Homo sapiens

<400>  122
tttaaggcta ggttcttccc aag                                               23


<210>  123
<211>  27
<212>  DNA
<213>  Homo sapiens

<400>  123
gactcttggt ctacaggtta tgaactc                                           27


<210>  124
<211>  20
<212>  DNA
<213>  Homo sapiens

<400>  124
tcaacacaag ctcctcatgg                                                   20


<210>  125
<211>  19
<212>  DNA
<213>  Homo sapiens

<400>  125
gacacacgac cgattcagc                                                    19


<210>  126
<211>  18
<212>  DNA
<213>  Homo sapiens

<400>  126
cacaggcatc ttagcgca                                                     18


<210>  127
<211>  18
<212>  DNA
<213>  Homo sapiens

<400>  127
ccaccaatgc cgagtttc                                                     18

<210>  128
<211>  18
<212>  DNA
<213>  Homo sapiens

<400>  128
cgatggctgg agcacttt                                                    18


<210>  129
<211>  19
<212>  DNA
<213>  Homo sapiens

<400>  129
catggagtga tttggcacc                                                   19


<210>  130
<211>  20
<212>  DNA
<213>  Homo sapiens

<400>  130
tgtgcttagt gggctgtcac                                                  20


<210>  131
<211>  23
<212>  DNA
<213>  Homo sapiens

<400>  131
ctgtgctaag taatcttcca cgg                                              23


<210>  132
<211>  20
<212>  DNA
<213>  Homo sapiens

<400>  132
aaagtgttgt tcccagcgag                                                  20


<210>  133
<211>  23
<212>  DNA
<213>  Homo sapiens

<400>  133
tgcctctgaa gatggttttc taa                                              23


<210>  134
<211>  19
<212>  DNA
<213>  Homo sapiens

<400>  134
tgttaagcat ttggcgcat                                                   19


<210>  135
<211>  22
<212>  DNA
<213>  Homo sapiens

<400>  135
gctttctgtg tagttgggga ct                                                22

<210>  136
<211>  21
<212>  DNA
<213>  Homo sapiens

<400>  136
gtaatccaca tgggcaacat t                                                 21

<210>  137
<211>  22
<212>  DNA
<213>  Homo sapiens

<400>  137
gtccccttaa atacacggat ga                                                22

<210>  138
<211>  19
<212>  DNA
<213>  Homo sapiens

<400>  138
aaggccacat agagacggc                                                    19

<210>  139
<211>  19
<212>  DNA
<213>  Homo sapiens

<400>  139
gcatcagtgg gaggcaata                                                    19

<210>  140
<211>  19
<212>  DNA
<213>  Homo sapiens

<400>  140
ggtcgtcacc aaacacctg                                                    19

<210>  141
<211>  19
<212>  DNA
<213>  Homo sapiens

<400>  141
ttaggcccct ctcatggac                                                    19

<210>  142
<211>  20
<212>  DNA
<213>  Homo sapiens

<400>  142
tctcatggtt tccttggacc                                                   20

<210>  143

<211>  21
<212>  DNA
<213>  Homo sapiens

<400>  143
acatctattg gaccttcgca a                                                21


<210>  144
<211>  18
<212>  DNA
<213>  Homo sapiens

<400>  144
ctctgtgggg ctgacacc                                                    18


<210>  145
<211>  20
<212>  DNA
<213>  Homo sapiens

<400>  145
aggaagcact gagccatttg                                                  20


<210>  146
<211>  19
<212>  DNA
<213>  Homo sapiens

<400>  146
aggacccctc gtgtgactc                                                   19


<210>  147
<211>  20
<212>  DNA
<213>  Homo sapiens

<400>  147
acctgctctt gggacacagt                                                  20


<210>  148
<211>  23
<212>  DNA
<213>  Homo sapiens

<400>  148
gaaaaggtga tggtgctttc taa                                              23


<210>  149
<211>  23
<212>  DNA
<213>  Homo sapiens

<400>  149
actaaatgca cagggatctc aaa                                              23


<210>  150
<211>  23
<212>  DNA
<213>  Homo sapiens

<400>  150

aaaggactca gggaagtacg ttt                                                23

<210>  151
<211>  24
<212>  DNA
<213>  Homo sapiens

<400>  151
gatactcctc ctatccaggt tgaa                                               24

<210>  152
<211>  22
<212>  DNA
<213>  Homo sapiens

<400>  152
aactgctgaa gataaaatcg cc                                                 22

<210>  153
<211>  19
<212>  DNA
<213>  Homo sapiens

<400>  153
tccctccatt gtctgaacg                                                     19

<210>  154
<211>  22
<212>  DNA
<213>  Homo sapiens

<400>  154
ttcccgttta tattggctaa cg                                                 22

<210>  155
<211>  20
<212>  DNA
<213>  Homo sapiens

<400>  155
tgtgctattc ttgacgctcg                                                    20

<210>  156
<211>  20
<212>  DNA
<213>  Homo sapiens

<400>  156
gtaaggccag gatttggtga                                                    20

<210>  157
<211>  21
<212>  DNA
<213>  Homo sapiens

<400>  157
tctgcctgcg tatgttagag g                                                  21

<210>  158
<211>  22

<212>  DNA
<213>  Homo sapiens

<400>  158
cacaggggca attagagaag tt                                              22


<210>  159
<211>  21
<212>  DNA
<213>  Homo sapiens

<400>  159
gccatgactg cataagtctc c                                               21


<210>  160
<211>  21
<212>  DNA
<213>  Homo sapiens

<400>  160
cgtttggttg tagtgccaac t                                               21


<210>  161
<211>  18
<212>  DNA
<213>  Homo sapiens

<400>  161
ggaccaagga gggcactt                                                   18


<210>  162
<211>  22
<212>  DNA
<213>  Homo sapiens

<400>  162
catgacccca tactgtcttc aa                                              22


<210>  163
<211>  26
<212>  DNA
<213>  Homo sapiens

<400>  163
catctattga ggactttacg acattg                                          26


<210>  164
<211>  19
<212>  DNA
<213>  Homo sapiens

<400>  164
tggctgagaa aacacctgg                                                  19


<210>  165
<211>  24
<212>  DNA
<213>  Homo sapiens

<400>  165
cactgagaga tccaaggcta gttt                                            24

<210> 166
<211> 23
<212> DNA
<213> Homo sapiens

<400> 166
tttctaaaga cattttcggc aac 23

<210> 167
<211> 18
<212> DNA
<213> Homo sapiens

<400> 167
agggaaatgt cagcgtgg 18

<210> 168
<211> 19
<212> DNA
<213> Homo sapiens

<400> 168
gcgatgtctc agggctaca 19

<210> 169
<211> 23
<212> DNA
<213> Homo sapiens

<400> 169
caaaatactg aacaggccca tta 23

<210> 170
<211> 19
<212> DNA
<213> Homo sapiens

<400> 170
gctgatttcc cctgcaagt 19

<210> 171
<211> 21
<212> DNA
<213> Homo sapiens

<400> 171
tattcggtgg gagaaacttg a 21

<210> 172
<211> 18
<212> DNA
<213> Homo sapiens

<400> 172
catctctggg cgaacgtc 18

<210> 173
<211> 20
<212> DNA

<213>  Homo sapiens

<400>  173
gtcgccattt tgctcctaga                                                    20


<210>  174
<211>  20
<212>  DNA
<213>  Homo sapiens

<400>  174
ctccgtgtca gcttagggac                                                    20


<210>  175
<211>  19
<212>  DNA
<213>  Homo sapiens

<400>  175
tgcaggacaa ggtttgagc                                                     19


<210>  176
<211>  24
<212>  DNA
<213>  Homo sapiens

<400>  176
gacagattat tgaagcactg ggta                                               24


<210>  177
<211>  19
<212>  DNA
<213>  Homo sapiens

<400>  177
acgtggcata acctcgtga                                                     19


<210>  178
<211>  20
<212>  DNA
<213>  Homo sapiens

<400>  178
aagcccaata ggaagcgtct                                                    20


<210>  179
<211>  20
<212>  DNA
<213>  Homo sapiens

<400>  179
cccaggatgt cactaccgat                                                    20


<210>  180
<211>  22
<212>  DNA
<213>  Homo sapiens

<400>  180
ctctcttccc agaggtcaac tg                                                 22

<210> 181
<211> 19
<212> DNA
<213> Homo sapiens

<400> 181
acggacatga acccaaggt 19

<210> 182
<211> 19
<212> DNA
<213> Homo sapiens

<400> 182
cctgtatggt ttgccgaga 19

<210> 183
<211> 22
<212> DNA
<213> Homo sapiens

<400> 183
agatatgcag agggcaacat ct 22

<210> 184
<211> 18
<212> DNA
<213> Homo sapiens

<400> 184
tcccacagaa ccaccgtt 18

<210> 185
<211> 21
<212> DNA
<213> Homo sapiens

<400> 185
ctgaaagcaa gtgggcttag a 21

<210> 186
<211> 20
<212> DNA
<213> Homo sapiens

<400> 186
catccccagc agtgaagttt 20

<210> 187
<211> 24
<212> DNA
<213> Homo sapiens

<400> 187
agaggagact gttactatgg cacc 24

<210> 188
<211> 18
<212> DNA
<213> Homo sapiens

<400>  188
ccttctgcat tccggcta                                                    18

<210>  189
<211>  21
<212>  DNA
<213>  Homo sapiens

<400>  189
tggggtcaca tagaagtcct g                                                21

<210>  190
<211>  20
<212>  DNA
<213>  Homo sapiens

<400>  190
atttgtcatt tggggacagc                                                  20

<210>  191
<211>  21
<212>  DNA
<213>  Homo sapiens

<400>  191
gtgcttcact gggtttctca a                                                21

<210>  192
<211>  21
<212>  DNA
<213>  Homo sapiens

<400>  192
tcaaaggcat caaccagatt t                                                21

<210>  193
<211>  19
<212>  DNA
<213>  Homo sapiens

<400>  193
ttctgacaag aggctgcgt                                                   19

<210>  194
<211>  23
<212>  DNA
<213>  Homo sapiens

<400>  194
gtaatccacg gaacacattt agc                                              23

<210>  195
<211>  21
<212>  DNA
<213>  Homo sapiens

<400>  195
ccgatgttgt atggacactc c                                                21

<210> 196
<211> 18
<212> DNA
<213> Homo sapiens

<400> 196
tgcacaccac tcttgcgt 18


<210> 197
<211> 18
<212> DNA
<213> Homo sapiens

<400> 197
accacttcga gacaggcg 18


<210> 198
<211> 19
<212> DNA
<213> Homo sapiens

<400> 198
tgctctgtga gggtggaaa 19


<210> 199
<211> 18
<212> DNA
<213> Homo sapiens

<400> 199
aggtgtatcc tgcgtccg 18


<210> 200
<211> 19
<212> DNA
<213> Homo sapiens

<400> 200
tggcttgcta aggggagtc 19


<210> 201
<211> 19
<212> DNA
<213> Homo sapiens

<400> 201
ggcagaatat gtgggagca 19


<210> 202
<211> 19
<212> DNA
<213> Homo sapiens

<400> 202
ttcagaggaa tcacacggc 19


<210> 203
<211> 18
<212> DNA
<213> Homo sapiens

<400>  203
caggcgtctt caaccagg                                                    18

<210>  204
<211>  18
<212>  DNA
<213>  Homo sapiens

<400>  204
gacgttcagg cccaactg                                                    18

<210>  205
<211>  25
<212>  DNA
<213>  Homo sapiens

<400>  205
aaatctattc caacagggac cttag                                            25

<210>  206
<211>  24
<212>  DNA
<213>  Homo sapiens

<400>  206
ttttacatct ggctaaaggg actc                                             24

<210>  207
<211>  18
<212>  DNA
<213>  Homo sapiens

<400>  207
acacacgacg aagccgtt                                                    18

<210>  208
<211>  20
<212>  DNA
<213>  Homo sapiens

<400>  208
tctgtcccaa gcctcagttt                                                  20

<210>  209
<211>  21
<212>  DNA
<213>  Homo sapiens

<400>  209
acataccttg ggagaacgct t                                                21

<210>  210
<211>  23
<212>  DNA
<213>  Homo sapiens

<400>  210
tttcctactg agaccacatg ctt                                              23

<210>  211

<211>  19
<212>  DNA
<213>  Homo sapiens

<400>  211
agtgaaccaa gggtgagcc                                                    19


<210>  212
<211>  19
<212>  DNA
<213>  Homo sapiens

<400>  212
aatggagaat cacgggcat                                                    19


<210>  213
<211>  18
<212>  DNA
<213>  Homo sapiens

<400>  213
gtttgcacgc atgactcg                                                     18


<210>  214
<211>  24
<212>  DNA
<213>  Homo sapiens

<400>  214
gaaaggctga tataacgaaa ttgg                                              24


<210>  215
<211>  21
<212>  DNA
<213>  Homo sapiens

<400>  215
ccctcaccac gtatcatttt g                                                 21


<210>  216
<211>  20
<212>  DNA
<213>  Homo sapiens

<400>  216
tgcaattatg ggctctgtga                                                   20


<210>  217
<211>  23
<212>  DNA
<213>  Homo sapiens

<400>  217
aaggggctga ctaattgtag gtc                                               23


<210>  218
<211>  18
<212>  DNA
<213>  Homo sapiens

<400>  218

ctgccaactc tgacgggt 18

<210> 219
<211> 19
<212> DNA
<213> Homo sapiens

<400> 219
gggatgcagg gctagtttc 19

<210> 220
<211> 23
<212> DNA
<213> Homo sapiens

<400> 220
caaaatgggt ctctcagtgc tac 23

<210> 221
<211> 19
<212> DNA
<213> Homo sapiens

<400> 221
gtgcaaactg aggccattg 19

<210> 222
<211> 23
<212> DNA
<213> Homo sapiens

<400> 222
tggtagaaca agaggttcct tga 23

<210> 223
<211> 24
<212> DNA
<213> Homo sapiens

<400> 223
agtttgttag gattttcaca ccgt 24

<210> 224
<211> 22
<212> DNA
<213> Homo sapiens

<400> 224
gcagacatta accctcatag gc 22

<210> 225
<211> 20
<212> DNA
<213> Homo sapiens

<400> 225
ggtggatgct tctgcctaaa 20

<210> 226
<211> 20

<212>  DNA
<213>  Homo sapiens

<400>  226
ttatgggtgc caagatagcc                                                    20


<210>  227
<211>  23
<212>  DNA
<213>  Homo sapiens

<400>  227
gaagagtcct tgtggttctc aaa                                                23


<210>  228
<211>  20
<212>  DNA
<213>  Homo sapiens

<400>  228
gggaagttgt aagcctgtgg                                                    20


<210>  229
<211>  21
<212>  DNA
<213>  Homo sapiens

<400>  229
agacccctca tagtggcaat c                                                  21


<210>  230
<211>  21
<212>  DNA
<213>  Homo sapiens

<400>  230
gaattacaca ccctccgtga a                                                  21


<210>  231
<211>  18
<212>  DNA
<213>  Homo sapiens

<400>  231
aaagcactcg acaaccgc                                                      18


<210>  232
<211>  19
<212>  DNA
<213>  Homo sapiens

<400>  232
gagaagctag tccccagcg                                                     19


<210>  233
<211>  19
<212>  DNA
<213>  Homo sapiens

<400>  233
tgagaacagt gccaggagc                                                     19

<210> 234
<211> 26
<212> DNA
<213> Homo sapiens

<400> 234
ggaataagga gtgtccatca taaaag 26

<210> 235
<211> 23
<212> DNA
<213> Homo sapiens

<400> 235
ctgtgaatag ctcgaaagcc tac 23

<210> 236
<211> 20
<212> DNA
<213> Homo sapiens

<400> 236
aagctgtctt aaatgcccca 20

<210> 237
<211> 26
<212> DNA
<213> Homo sapiens

<400> 237
taatcttgtt ctaagttccc tcgaat 26

<210> 238
<211> 20
<212> DNA
<213> Homo sapiens

<400> 238
cctctgaatc ctcccaagtg 20

<210> 239
<211> 23
<212> DNA
<213> Homo sapiens

<400> 239
tgtggttagg taaagaggac tgc 23

<210> 240
<211> 20
<212> DNA
<213> Homo sapiens

<400> 240
cagggatcta ccccagttga 20

## Beschreibung

Die vorliegende Erfindung betrifft ein Verfahren zur parallelen Amplifikation von wenigstens zwei verschiedenen Nukleinsäureabschnitten aus einer Nukleinsäure enthaltenden Probe.

Üblicherweise erfolgt die Amplifikation von Nukleinsäureabschnitten aus einer Nukleinsäure enthaltenden Probe mittels einer Polymerasekettenreaktion (PCR). Sollen mehrere Nukleinsäureabschnitte parallel amplifiziert werden, wird die PCR vorzugsweise als Multiplex-PCR ausgeführt, bei der der Probe mehrere Primerpaare zugegeben werden, so dass die parallele Amplifikation in einem Probenansatz erfolgen kann. Wenn die Ausgangsprobe jedoch wenig Nukleinsäure enthält, beispielsweise weniger als 10 pg, liegt die Wahrscheinlichkeit dafür, in einer Einschritt-PCR ein PCR-Produkt zu erhalten, bei erfahrungsgemäß deutlich unterhalb von 100 %. Zudem besteht bei der Durchführung einer Einschritt-PCR immer das Risiko, zusätzlich zu oder anstelle des gewünschten PCR-Produkts Artefakte zu erhalten.

Um die Sensitivität und die Spezifität der PCR insbesondere auch mit wenig Nukleinsäure enthaltenden Ausgangsproben zu erhöhen, sind bereits Zwei-Schritt PCR's, wie beispielsweise die WGA und die nested PCR, vorgeschlagen worden. Bei diesen Verfahren werden jeweils zwei Amplifikationsreaktionen nacheinander durchgeführt werden, wobei in der zweiten PCR eine Teilmenge des Produkts der ersten PCR als Ausgangsmaterial eingesetzt wird.

Bei der WGA ("Whole Genome Amplification" bzw. "Vervielfältigung des gesamten Genoms") wird zunächst eine PCR unter Einsatz von randomisierten, d.h. unspezifischen, Primern durchgeführt, um möglichst die gesamte in der Ausgangsprobe vorliegende Nukleinsäure zu amplifizieren, bevor mit dem Produkt der ersten PCR anschließend eine zweite PCR unter Einsatz von für die zu amplifizierenden Zielabschnitte spezifischen Primerpaaren durchgeführt wird. Ein auf der WGA basierendes Verfahren ist beispielsweise in der US 6,365,375 B1 beschrieben worden. Bei diesem Verfahren wird für die beiden PCR's jeweils eine Mischung aus zwei DNA-Polymerasen eingesetzt, wobei wenigstens eine der beiden DNA-Polymerasen eine 3'-5'-Exonukleaseaktivität aufweist.

Allerdings weist die WGA den Nachteil auf, dass bei der ersten, unspezifischen PCR, insbesondere wenn in der Ausgangsprobe nur wenig Nukleinsäure vorliegt, nicht die gesamte Nukleinsäure amplifiziert wird, so dass unter Umständen bei der zweiten PCR keine die Zielsequenzen umfassende Nukleinsäure bzw. nur ungenügende Mengen von die Zielsequenzen umfassender Nukleinsäure vorliegen.

Im Unterschied dazu werden bei der nested PCR ("geschachtelten PCR") sowohl in der ersten als auch in der nachfolgenden zweiten PCR jeweils für die zu amplifizierenden Zielsequenzen spezifische Primer eingesetzt. Dabei werden in der ersten PCR und in der zweiten PCR für jede zu amplifizierende Zielsequenz jeweils unterschiedliche Primerpaare eingesetzt, wobei die Primerbindungsstellen für das in der zweiten PCR eingesetzte Primerpaar jeweils stromabwärts der Primerbindungsstellen für das in der ersten PCR für denselben Zielabschnitt eingesetzte Primerpaar liegen, so dass bei der zweiten PCR für jede Zielsequenz ein kürzeres PCR-Produkt erhalten wird als bei der ersten PCR. Entsprechende Verfahren sind beispielsweise von F. Ray et al. in Molecular Human Reproduction (2001), Band 7, Nummer 5, Seiten 489 bis 494 und von A. Girardet et al. in Molecular Human Reproduction (2003), Band 9, Nummer 7, Seiten 421 bis 427 beschrieben worden. Ein Nachteil der auf nested PCR basierenden Verfahren liegt in der Verwendung von unterschiedlichen Primerpaaren für die erste und die zweite PCR. Dies bedingt zum einen einen hohen Aufwand für den Entwurf als auch für die Synthese der benötigten Primer, was insbesondere in dem Fall, dass mehrere Zielsequenzen, beispielsweise 100 unterschiedliche Zielsequenzen, parallel amplifiziert werden sollen, zu einem unverhältnismäßig hohem Aufwand und zu hohen Kosten führt. Zum anderen müssen die erste und die zweite PCR bei unterschiedlichen Temperaturprofilen durchgeführt werden, um die Interaktion der ersten Primerpaare während der zweiten PCR mit den zweiten Primerpaaren zu vermeiden. Ferner bedingt der Einsatz von zwei verschiedenen Primerpaaren pro Zielsequenz immer das Risiko, dass in der zweiten PCR Primer eingesetzt werden, welche nicht zu den in der ersten PCR eingesetzten Primern passen bzw. mit diesen inkompatibel sind.

Aufgabe der vorliegenden Erfindung ist daher die Bereitstellung eines Verfahrens zur parallelen Amplifikation von mehreren unterschiedlichen Nukleinsäureabschnitten aus einer Nukleinsäure enthaltenden Probe, welches schnell und einfach durchgeführt werden kann, welches den Einsatz möglichst weniger unterschiedlicher Primerpaare erfordert und welches auch bei Ausgangsmaterial, welches nur wenig Nukleinsäure enthält, wie beispielsweise einer eine Zelle enthaltenden Ausgangsprobe, zu zuverlässigen Ergebnissen führt.

Erfindungsgemäß wird diese Aufgabe gelöst durch ein Verfahren, welches die nachfolgenden Schritte umfasst:
a) Bereitstellen einer Nukleinsäure enthaltenden Probe,
b) Zugabe von wenigstens zwei Primerpaaren zu der Probe, wobei jedes der wenigstens zwei Primerpaare für einen der wenigstens zwei verschiedenen, zu amplifizierenden Nukleinsäureabschnitte spezifisch ist,
c) Durchführen einer PCR-Reaktion mit der in dem Schritt b) erhaltenen Probe,
d) Aufteilen der in dem Schritt c) erhaltenen Probe in wenigstens zwei Teilmengen und Zugabe von jeweils wenigstens einem Primerpaar zu jeder der Teilmengen, wobei jedes den Teilmengen zugesetzte Primerpaar mit jeweils einem der in dem Schritt b) zugegebenen Primerpaare identisch ist und sich das/die den einzelnen Teilmengen zugegebene(n) Primerpaar(e) jeweils von dem/den den anderen Teilmengen zugegebenen Primerpaar(en) unterscheidet/unterscheiden, sowie
e) Durchführen einer PCR-Reaktion mit den in Schritt d) erhaltenen Teilmengen.

Die vorliegende Erfindung basiert auf der überraschenden Erkenntnis, dass eine Zweischritt-PCR, bei der nacheinander zwei PCR's unter Verwendung derselben Primerpaare durchgeführt werden, eine hervorragende Sensitivität sowie eine ausgezeichnete Spezifität aufweist. Dabei dient die erste PCR der Prä- bzw. Voramplifikation der zu amplifizierenden Nukleinsäureabschnitte, d.h. dazu, um die Anzahl der zu amplifizierenden Nukleinsäureabschnitte bezogen auf die Anzahl der nicht zu amplifizierenden Nukleinsäureabschnitte zu erhöhen, um bei der zweiten PCR möglichst wenig störende "Hintergrund"-DNA vorliegen zu haben. Aufgrund seiner hervorragenden Sensitivität sowie seiner ausgezeichneten Spezifität ist das erfindungsgemäße Verfahren insbesondere zur parallelen Amplifikation einer Vielzahl von verschiedenen Nukleinsäureabschnitten, beispielsweise von 100 verschiedenen Nukleinsäureabschnitten, aus einer Nukleinsäure enthaltenden Probe geeignet und insbesondere auch zur parallelen Amplifikation einer Vielzahl von verschiedenen Nukleinsäureabschnitten aus einer Probe geeignet, welche sehr wenig Nukleinsäure enthält. Insbesondere eignet sich das erfindungsgemäße Verfahren zur parallelen Amplifikation von einer Vielzahl von verschiedenen Nukleinsäureabschnitten aus einer Einzelzelle. Da in den beiden PCR's des erfindungsgemäßen Verfahrens jeweils die gleichen Primerpaare eingesetzt werden, sind der Aufwand und die Kosten für das Entwerfen sowie das Synthetisieren der Primerpaare auf ein Minimum beschränkt und insbesondere können so zuverlässig nachteilige Primerinteraktionen vermieden werden oder zumindest auf ein Minimum reduziert werden. Da in dem erfindungsgemäßen Verfahren im Vergleich zu den aus dem Stand der Technik bekannten Zwei-schritt-PCR-Verfahren weniger Primerpaare eingesetzt werden, ist das erfindungsgemäße Verfahren insbesondere auch für kurze Zielsequenzen geeignet. Es ist bekannt, dass es bei kurzen Zielsequenzen schwierig sein kann, passende Primersequenzen aufzufinden, was naturgemäß bei den aus dem Stand der Technik bekannten Verfahren, bei denen pro Zielsequenz zwei verschiedene Primerpaare einzusetzen sind, problematisch sein kann. Dies wird durch das erfindungsgemäße Verfahren vermieden. Insbesondere kann so zuverlässig vermieden werden, dass in der zweiten PCR Primer eingesetzt werden, welche mit den in der ersten PCR eingesetzten Primern nicht kompatibel sind.

Wie vorstehend dargelegt, eignet sich das erfindungsgemäße Verfahren insbesondere zur parallelen Amplifikation von mehreren verschiedenen Nukleinsäureabschnitten aus einer geringe Mengen an Nukleinsäure enthaltenden Probe. Insbesondere eignet sich das erfindungsgemäße Verfahren zur parallelen Amplifikation von mehreren verschiedenen Nukleinsäureabschnitten aus einer Probe, welche weniger als 10 ng, vorzugsweise weniger als 1 ng, besonders bevorzugt weniger als 100 pg und ganz besonders bevorzugt weniger als 10 pg Nukleinsäure enthält.

Dementsprechend ist das erfindungsgemäße Verfahren insbesondere auch zur parallelen Amplifikation von mehreren verschiedenen Nukleinsäureabschnitten aus einer Probe geeignet, welche weniger als 100 Zellen, vorzugsweise weniger als 10 Zellen, besonders bevorzugt weniger als 3 Zellen und ganz besonders bevorzugt genau eine Zelle enthält.

Bezüglich der Quelle der als Ausgangsmaterial eingesetzten Zelle(n) ist das erfindungsgemäße Verfahren nicht beschränkt, so dass alle menschlichen und tierischen Zellen sowie alle Zellen von Mikroorganismen, beispielsweise Bakterienzellen, Hefezellen, Pilzzellen und dergleichen, eingesetzt werden können. Vorzugsweise enthält die in dem Verfahrensschritt a) bereitgestellte Probe humane Zellen, wobei insbesondere mit einer Probe, welche genau eine humane Zelle, beispielsweise einen Polkörper, enthält, gute Ergebnisse erhalten werden.

Aufgrund der Tatsache, dass in der zweiten PCR nur Primer eingesetzt werden, welche bereits bei der ersten PCR eingesetzt worden sind, eignet sich das erfindungsgemäße Verfahren zur parallelen Amplifikation einer Vielzahl verschiedener Zielsequenzen aus einer Nukleinsäure enthaltenden Probe. Vorzugsweise werden der Probe in dem Verfahrensschritt b) 5 bis 500, besonders bevorzugt 10 bis 250 und ganz besonders bevorzugt zwischen 20 und 100 verschiedene Primerpaare zugesetzt. Vorzugsweise entspricht die Anzahl der eingesetzten Primerpaare der Anzahl der zu amplifizierenden Zielsequenzen. Mithin eignet sich das erfindungsgemäße Verfahren insbesondere zur Amplifikation von 5 bis 500, besonders bevorzugt 10 bis 250 und ganz besonders bevorzugt 20 bis 100 verschiedenen Zielsequenzen.

In Weiterbildung des Erfindungsgedankens wird vorgeschlagen, dass die Gesamtanzahl der den Teilmengen in dem Schritt d) zugegebenen Primerpaare der Anzahl der der Probe in dem Schritt b) zugegebenen Primerpaare entspricht.

Gemäß einer weiteren bevorzugten Ausführungsform der vorliegenden Erfindung werden jeder der wenigstens zwei Teilmengen in dem Verfahrensschritt d) jeweils 2 bis 20 Primerpaare, besonders bevorzugt jeweils 5 bis 15 Primerpaare und ganz besonders bevorzugt jeweils 8 bis 12 Primerpaare zugegeben. Indem jeder der wenigstens zwei Teilmengen wenigstens 2, vorzugsweise wenigstens 5, Primerpaare zugegeben werden, kann die Anzahl der verschiedenen Teilmengen, in welche die in dem Verfahrensschritt c) erhaltene Probe aufgeteilt wird, vergleichsweise gering gehalten werden, so dass in dem Verfahrensschritt e) nur vergleichsweise wenige verschiedene Proben einer PCR unterworfen werden müssen. Zudem wird dadurch, dass jeder der wenigstens zwei Teilmengen vorzugsweise maximal 20 Primerpaare zugegeben wird, erreicht, dass die Gesamtzahl der in einem PCR-Ansatz enthaltenen Primerpaare nicht so groß ist, dass sich die Primerpaare untereinander stören, so dass bei der in dem Verfahrensschritt e) durchgeführten PCR für jedes eingesetzte Primerpaar mit hoher Wahrscheinlichkeit ein PCR-Produkt erhalten wird.

Mithin hängt die konkrete Anzahl der Teilmengen, in welche die in dem Verfahrensschritt c) erhaltene Probe aufgeteilt wird, in erster Linie von der eingesetzten Anzahl an Primerpaaren bzw. der konkreten Anzahl an zu amplifizierenden Nukleinsäureabschnitten ab. Vorzugsweise wird die Probe in dem Verfahrensschritt d) in 2 bis 250, besonders bevorzugt in 5 bis 125 und ganz besonders bevorzugt in 8 bis 50 Teilmengen aufgeteilt.

Um eine gute Amplifikation der zu amplifizierenden Nukleinsäureabschnitte zu erreichen, wird die Probe vor der Durchführung der ersten PCR gemäß dem Verfahrensschritt b), d.h. vor oder nach der Zugabe der Primerpaare, vorzugsweise durch den Zusatz wenigstens einer DNA-Polymerase und ggf. wenigstens einer aus der aus pH-Puffersubstanzen, Salzen, Wasser und weiteren üblichen PCR-Zusatzstoffen bestehenden Gruppe ausgewählten Verbindung auf die für die Durchführung der PCR optimalen Bedingungen eingestellt. Zu diesem Zweck können alle dem Fachmann bekannten DNA-Polymerasen, wie beispielsweise Taq-DNA-Polymerase, Pfu-DNA-Polymerase oder dergl., sowie alle weiteren dem Fachmann bekannten Zusatz- und Hilfsstoffe, wie beispielsweise kommerziell erhältliche PCR-Puffer, eingesetzt werden.

In Weiterbildung des Erfindungsgedankens wird vorgeschlagen, die PCR-Reaktion in dem Verfahrensschritt c) mit nicht mehr als 25 Zyklen, vorzugsweise mit 5 bis 20 Zyklen und besonders bevorzugt mit 10 bis 15 Zyklen durchzuführen. Dadurch wird erreicht, dass bei der ersten PCR nur die gewünschte Präamplifikation der zu amplifizierenden Nukleinsäureabschnitte, d.h. eine Erhöhung des Verhältnisses von zu amplifizierenden Nukleinsäureabschnitten zu nicht zu amplifizierenden Nukleinsäureabschnitten, erreicht wird, ohne dass jedoch in einem signifikanten Umfang störende, auf die Anwesenheit der nicht zu amplifizierenden Nukleinsäureabschnitte zurückzuführende Konkurrenz- bzw. Nebenreaktionen auftreten. Nach der Präamplifikation gemäß dem Verfahrensschritt c) liegen somit auch die zu amplifizierenden Nukleinsäuresequenzen in einer sehr geringen Menge von in der Regel weniger als 100 pg vor, welche jedoch ausreichend ist, um die Aufteilung der Probe in mehrere Teilmengen mit jeweils genügend Menge an Nukleinsäurezielsequenzen für eine Amplifikation in der zweiten PCR zu ermöglichen.

Vorzugsweise werden auch die Teilmengen vor der Durchführung der zweiten PCR gemäß dem Verfahrensschritt e), d.h. vor oder nach der in dem Verfahrensschritt d) erfolgenden Zugabe der Primer, bevorzugt durch den Zusatz wenigstens einer DNA-Polymerase und ggf. wenigstens einer aus der aus pH-Puffersubstanzen, Salzen, Wasser und weiteren üblichen PCR-Zusatzstoffen bestehenden Gruppe ausgewählten Verbindung auf die für die Durchführung der PCR optimalen Bedingungen eingestellt.

Um in der zweiten PCR gemäß dem Verfahrensschritt e) in den einzelnen Teilmengen jeweils nur die den den jeweiligen Teilmengen in dem Verfahrensschritt d) zugegebenen Primerpaaren entsprechenden Nukleinsäureabschnitte zu amplifizieren, d.h. um einen störenden Einfluss derjenigen Primerpaare, welche der Probe in dem Verfahrensschritt b) für die erste PCR zugegeben worden sind, welche der entsprechenden Teilmenge aber nicht mehr in dem Verfahrensschritt d) zugegeben worden sind, in der zweiten PCR zu vermeiden, wird in Weiterbildung des Erfindungsgedankens vorgeschlagen, die Teilmengen vor der Durchführung der PCR gemäß dem Verfahrensschritt e) um den Faktor 2 bis 250, vorzugsweise um den Faktor 5 bis 125 und besonders bevorzugt um den Faktor 8 bis 50 zu verdünnen. Dies kann durch den Zusatz der zuvor erwähnten, für die Einstellung der optimalen PCR-Bedingungen notwendigen Zusatz- und Hilfsstoffe, insbesondere durch Wasser, erreicht werden.

Gemäß einer weiteren bevorzugten Ausführungsform der vorliegenden Erfindung wird die PCR-Reaktion in dem Schritt e) mit wenigstens 20 Zyklen und vorzugsweise mit 20 bis 40 Zyklen durchgeführt. Auf diese Weise kann eine quantitativ ausreichende Amplifikation der zu amplifizierenden Nukleinsäureabschnitte in den Teilmengen erreicht werden.

Bei den zu amplifizierenden Nukleinsäureabschnitten kann es sich um alle kodierenden und nicht-kodierenden Nukleinsäuresequenzen handeln. Insbesondere kann jeder der wenigstens zwei zu amplifizierenden Nukleinsäureabschnitte, unabhängig voneinander, ein Gen, ein Genabschnitt oder eine nicht-kodierende Nukleinsäuresequenz sein.

Gemäß einer besonders bevorzugten Ausführungsform betrifft die vorliegende Erfindung ein Verfahren zur Amplifikation von 5 bis 500 verschiedenen Nukleinsäureabschnitten aus einer Nukleinsäure enthaltenden Probe, welches die nachfolgenden Schritte umfasst:
a) Bereitstellen einer Nukleinsäure enthaltenden Probe, welche weniger als 10 pg Nukleinsäure und/oder genau eine Zelle enthält,
b) Zugabe von wenigstens 5 bis 500 Primerpaaren zu der Probe, wobei jedes der 5 bis 500 Primerpaare für einen der 5 bis 500 verschiedenen, zu amplifizierenden Nukleinsäureabschnitte spezifisch ist,
c) Durchführen einer PCR-Reaktion mit der in dem Schritt b) erhaltenen Probe,
d) Aufteilen der in dem Schritt c) erhaltenen Probe in wenigstens zwei Teilmengen und Zugabe von jeweils 2 bis 20 Primerpaaren zu jeder der Teilmengen, wobei jedes den Teilmengen zugesetzte Primerpaar mit jeweils einem der in dem Schritt b) zugegebenen Primerpaare identisch ist und sich die den einzelnen Teilmengen zugegebenen Primerpaare jeweils von den den anderen Teilmengen zugegebenen Primerpaaren unterscheiden, sowie
e) Durchführen einer PCR-Reaktion mit den in Schritt d) erhaltenen Teilmengen.

Ein weiterer Gegenstand der vorliegenden Erfindung ist ein Verfahren zur Bestimmung des Genotyps eines oder mehrerer Individuen aus einer Nukleinsäure enthaltenden biologischen Probe, welches die nachfolgenden Schritte umfasst:
a) Bereitstellen einer Nukleinsäure enthaltenden Probe,
b) Zugabe von wenigstens zwei Primerpaaren zu der Probe, wobei jedes der wenigstens zwei Primerpaare für einen von wenigstens zwei verschiedenen, zu amplifizierenden Nukleinsäureabschnitte spezifisch ist,
c) Durchführen einer PCR-Reaktion mit der in dem Schritt b) erhaltenen Probe,
d) Aufteilen der in dem Schritt c) erhaltenen Probe in wenigstens zwei Teilmengen und Zugabe von jeweils wenigstens einem Primerpaar zu jeder der Teilmengen, wobei jedes den Teilmengen zugesetzte Primerpaar mit jeweils einem der in dem Schritt b) zugegebenen Primerpaare identisch ist und sich das/die den einzelnen Teilmengen zugegebene(n) Primerpaar(e) jeweils von dem/den den anderen Teilmengen zugegebenen Primerpaar(en) unterscheidet/unterscheiden,
e) Durchführen einer PCR-Reaktion mit den in Schritt d) erhaltenen Teilmengen und
f) Charakterisierung der in Schritt e) erhaltenen PCR-Produkte.

Vorzugsweise umfasst die in dem Schritt f) durchgeführte Charakterisierung der in dem Schritt e) erhaltenen PCR-Produkte die Bestimmung, mit wie vielen und/oder mit welchen der eingesetzten Primerpaare ein PCR-Produkt erhalten worden ist.

Die Charakterisierung der PCR-Produkte kann beispielsweise mittels Gelelektrophorese, mittels Kapillarelektrophorese, mittels Sequenzierung und/oder mittels einer Hybridisierungstechnik auf einem DNA-Array, einem Bead-System oder einer anderen optischen, elektrischen oder elektrochemischen Messung erfolgen.

Nachfolgend wird die vorliegende Erfindung anhand eines beispielhaften, die vorliegende Erfindung jedoch nicht beschränkenden Beispiels näher erläutert.

### Beispiel

Es wurde ein Verfahren zur Amplifikation von 120 verschiedenen Nukleinsäureabschnitten aus einer 100 pg humane, männliche DNA enthaltenden biologischen Probe basierend auf dem zuvor beschriebenen Zweischritt-PCR-Verfahren durchgeführt. Dabei wurde zunächst nach Zugabe von 120 Primerpaaren, d.h. von 240 Primern, von Taq-Polymerase und von PCR-Puffer zu der biologischen Probe eine Multiplex-PCR durchgeführt, bevor die so erhaltene Probe in 12 Teilmengen aufgeteilt wurde. Die Nukleinsäuresequenzen der eingesetzten Primer (SEQ ID No. 1 bis SEQ ID No. 240) sind in dem beigefügten Sequenzprotokoll wiedergegeben. Dabei wurden jeweils aufeinander folgende Primer als ein Primerpaar eingesetzt, d.h. die Nukleinsäuresequenzen SEQ ID No. 1 und SEQ ID No. 2 wurden als erstes Primerpaar eingesetzt, die Nukleinsäuresequenzen SEQ ID No. 3 und SEQ ID No. 4 wurden als zweites Primerpaar eingesetzt usw.

Anschließend wurden jeder der 12 Teilmengen jeweils 10 Primerpaare, PCR-Puffer und Taq-Polymerase zugesetzt, bevor die einzelnen Teilmengen einer zweiten PCR unterzogen wurden. Jedes den einzelnen Teilmengen zugesetzte Primerpaar entsprach einem der in der ersten PCR zugegebenen Primerpaare, wobei sich die den einzelnen Teilmengen zugegebenen Primerpaare jeweils von den den anderen Teilmengen zugegebenen Primerpaaren unterschieden. Anschließend wurde jeweils ein Aliquot jeder der 12 Teilmengen in jeweils eine Tasche eines Polyacrylamidgels aufgetragen, elektrophoretisch aufgetrennt und nach Silberfärbung detektiert.

Dabei wurden die einzelnen Reaktionsschritte wie folgt durchgeführt.

### Erste PCR

1µl einer DNA-Lösung enthaltend 100pg/µl humane, männliche DNA (Promega 9948) wurde auf die Reaktionsposition eines AmpliGrid® (Advalytix AG, München) pipettiert und bei 37 °C eintrocknen gelassen. Anschließend wurde zu der Probe auf die Reaktionsposition 1µl einer 120 Primerpaare enthaltenden Primermischung pipettiert und ebenfalls bei 37 °C eintrocknen gelassen. Dann wurde zu der Probe auf die Reaktionsposition 1 µl einer PCR-Mischung, welche 0,5 µm 2x Multiplex PCR Master Mix (Qiagen GmbH, Hilden), 0,005 µl Bromphenolblau (0,1 %), 0,06 µl 5x Q-Solution (Qiagen GmbH, Hilden) und 0,39 µl Wasser enthielt, zugegeben. Abschließend wurde der Reaktionsansatz mit 5 µl Sealing Solution (Advalytix AG, München) bedeckt und mit dem nachfolgenden Temperaturprofil einer PCR unterzogen:
- 10 Minuten bei 94 °C,
- 11 Zyklen mit 94 °C für 30 Sekunden, 58 °C für 90 Sekunden und 72 °C für 60 Sekunden, sowie
- 10 Minuten bei 72 °C.

### Zweite PCR

Auf 12 Reaktionspositionen eines AmpliGrid® wurden je 1 µl einer Primermischung mit jeweils 10 Primerpaaren pipettiert und bei 37 °C eintrocknen gelassen. Anschließend wurde das Reaktionsprodukt der ersten PCR mit 29 µl Wasser verdünnt und vermischt, bevor jeweils 1 µl der so erhaltenen Mischung auf jede der 12 Reaktionspositionen des AmpliGrid® aufgetragen und bei 37 °C eintrocknen gelassen wurde. Dann wurde zu der Probe auf jede der 12 Reaktionspositionen jeweils 1 µl einer PCR-Mischung, welche 0,5 µm 2x Multiplex PCR Master Mix (Qiagen GmbH, Hilden), 0,005 µl Bromphenolblau (0,1 %), 0,06 µl 5x Q-Solution (Qiagen GmbH, Hilden) und 0,39 µl Wasser enthielt, zugegeben. Abschließend wurde der Reaktionsansatz mit 5 µl Sealing Solution (Advalytix AG, München) bedeckt und mit dem nachfolgenden Temperaturprofil einer PCR unterzogen:
- 10 Minuten bei 94 °C,
- 35 Zyklen mit 94 °C für 30 Sekunden, 63 °C für 60 Sekunden und 72 °C für 60 Sekunden, sowie
- 10 Minuten bei 72 °C.

### Gelelektrophorese

Anschließend wurde jeweils eine Teilmenge jeder der 12 PCR-Produkte der zweiten PCR in eine Tasche eines Polyacrylamidgels pipetiert, elektrophoretisch aufgetrennt und nach Silberfärbung detektiert.

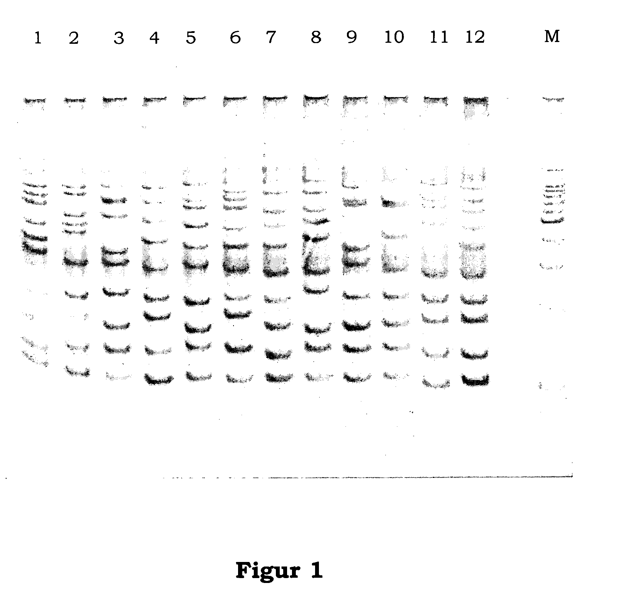

Eine Photographie des Polyacrylamidgels ist in der Figur 1 dargestellt. In der Figur 1 bezeichnen die Zahlen 1 bis 12 die Taschen für die einzelnen Teilmengen der zweiten PCR-Reaktion, wohingegen M einen Marker, nämlich eine 100 Basenpaar-Leiter, bezeichnet.

In jeder der Teilmengen der zweiten PCR wurden 9 bis 10 von jeweils 10 möglichen PCR-Produkten erhalten.

## Patentansprüche

1. Verfahren zur Amplifikation von wenigstens zwei verschiedenen Nukleinsäureabschnitten aus einer Nukleinsäure enthaltenden Probe umfassend die Schritte:
a) Bereitstellen einer Nukleinsäure enthaltenden Probe,
b) Zugabe von wenigstens zwei Primerpaaren zu der Probe, wobei jedes der wenigstens zwei Primerpaare für einen der wenigstens zwei verschiedenen, zu amplifizierenden Nukleinsäureabschnitte spezifisch ist,
c) Durchführen einer PCR-Reaktion mit der in dem Schritt b) erhaltenen Probe,
d) Aufteilen der in dem Schritt c) erhaltenen Probe in wenigstens zwei Teilmengen und Zugabe von jeweils wenigstens einem Primerpaar zu jeder der Teilmengen, wobei jedes den Teilmengen zugesetzte Primerpaar mit jeweils einem der in dem Schritt b) zugegebenen Primerpaare identisch ist und sich das/die den einzelnen Teilmengen zugegebene(n) Primerpaar(e) jeweils von dem/den den anderen Teilmengen zugegebenen Primerpaar(en) unterscheidet/unterscheiden, sowie
e) Durchführen einer PCR-Reaktion mit den in Schritt d) erhaltenen Teilmengen.

2. Verfahren nach Anspruch 1,
**dadurchgekennzeichnet, dass** die in Schritt a) bereitgestellte Probe weniger als 10 ng, vorzugsweise weniger als 1 ng, besonders bevorzugt weniger als 100 pg und ganz besonders bevorzugt weniger als 10 pg Nukleinsäure enthält.

3. Verfahren nach Anspruch 1 oder 2,
**dadurchgekennzeichnet, dass** die in Schritt a) bereitgestellte Probe weniger als 100 Zellen, vorzugsweise weniger als 10 Zellen, besonders bevorzugt weniger als 3 Zellen und ganz besonders bevorzugt genau eine Zelle enthält.

4. Verfahren nach Anspruch 3,
**dadurchgekennzeichnet, dass** die in Schritt a) bereitgestellte Probe genau eine humane Zelle, vorzugsweise einen Polkörper, enthält.

5. Verfahren nach zumindest einem der vorstehenden Ansprüche,
**dadurchgekennzeichnet,dass** der Probe in dem Schritt b) 5 bis 500, vorzugsweise 10 bis 250 und besonders bevorzugt zwischen 20 und 100 verschiedene Primerpaare zugesetzt werden.

6. Verfahren nach zumindest einem der vorstehenden Ansprüche,
**dadurchgekennzeichnet,dass** die Gesamtanzahl der den Teilmengen in dem Schritt d) zugegebenen Primerpaare der Anzahl der der Probe in dem Schritt b) zugegebenen Primerpaare entspricht.

7. Verfahren nach zumindest einem der vorstehenden Ansprüche,
**dadurchgekennzeichnet, dass** jeder der wenigstens zwei Teilmengen in dem Schritt d) jeweils 2 bis 20 Primerpaare, vorzugsweise jeweils 5 bis 15 Primerpaare und besonders bevorzugt 8 bis 12 Primerpaare zugegeben wird.

8. Verfahren nach zumindest einem der vorstehenden Ansprüche,
**dadurchgekennzeichnet, dass** die Probe in dem Schritt d) in 2 bis 250, vorzugsweise in 5 bis 125 und besonders bevorzugt in 8 bis 50 Teilmengen aufgeteilt wird.

9. Verfahren nach zumindest einem der vorstehenden Ansprüche,
**dadurchgekennzeichnet, dass** die PCR-Reaktion in dem Schritt c) mit nicht mehr als 25 Zyklen, vorzugsweise mit 5 bis 20 Zyklen und besonders bevorzugt mit 10 bis 15 Zyklen durchgeführt wird.

10. Verfahren nach zumindest einem der vorstehenden Ansprüche,
**dadurchgekennzeichnet, dass** die PCR-Reaktion in dem Schritt e) mit wenigstens 20 Zyklen und vorzugsweise mit 20 bis 40 Zyklen durchgeführt wird.

11. Verfahren zur Amplifikation von 5 bis 500 verschiedenen Nukleinsäureabschnitten aus einer Nukleinsäure enthaltenden Probe umfassend die Schritte:
a) Bereitstellen einer Nukleinsäure enthaltenden Probe, welche weniger als 10 pg Nukleinsäure und/oder genau eine Zelle enthält,
b) Zugabe von wenigstens 5 bis 500 Primerpaaren zu der Probe, wobei jedes der 5 bis 500 Primerpaare für einen der 5 bis 500 verschiedenen, zu amplifizierenden Nukleinsäureabschnitte spezifisch ist,
c) Durchführen einer PCR-Reaktion mit der in dem Schritt b) erhaltenen Probe,
d) Aufteilen der in dem Schritt c) erhaltenen Probe in wenigstens zwei Teilmengen und Zugabe von jeweils 2 bis 20 Primerpaaren zu jeder der Teilmengen, wobei jedes den Teilmengen zugesetzte Primerpaar mit jeweils einem der in dem Schritt b) zugegebenen Primerpaare identisch ist und sich die den einzelnen Teilmengen zugegebenen Primerpaare jeweils von den den anderen Teilmengen zugegebenen Primerpaaren unterscheiden, sowie
e) Durchführen einer PCR-Reaktion mit den in Schritt d) erhaltenen Teilmengen.

12. Verfahren zur Bestimmung des Genotyps eines oder mehrerer Individuen aus einer Nukleinsäure enthaltenden biologischen Probe umfassend die Schritte:
a) Bereitstellen einer Nukleinsäure enthaltenden Probe,
b) Zugabe von wenigstens zwei Primerpaaren zu der Probe, wobei jedes der wenigstens zwei Primerpaare für einen von wenigstens zwei verschiedenen, zu amplifizierenden Nukleinsäureabschnitte spezifisch ist,
c) Durchführen einer PCR-Reaktion mit der in dem Schritt b) erhaltenen Probe,
d) Aufteilen der in dem Schritt c) erhaltenen Probe in wenigstens zwei Teilmengen und Zugabe von jeweils wenigstens einem Primerpaar zu jeder der Teilmengen, wobei jedes den Teilmengen zugesetzte Primerpaar mit jeweils einem der in dem Schritt b) zugegebenen Primerpaare identisch ist und sich das/die den einzelnen Teilmengen zugegebene(n) Primerpaar(e) jeweils von dem/den den anderen Teilmengen zugegebenen Primerpaar(en) unterscheidet/unterscheiden,
e) Durchführen einer PCR-Reaktion mit den in Schritt d) erhaltenen Teilmengen und
f) Charakterisierung der in Schritt e) erhaltenen PCR-Produkte.

13. Verfahren Anspruch 12,
**dadurchgekennzeichnet, dass** die in dem Schritt f) durchgeführte Charakterisierung der in dem Schritt e) erhaltenen PCR-Produkte die Bestimmung umfasst, mit wie vielen und/oder mit welchen der eingesetzten Primerpaare ein PCR-Produkt erhalten worden ist.

14. Verfahren Anspruch 12 oder 13,
**dadurchgekennzeichnet, dass** die Charakterisierung der PCR-Produkte mittels Gelelektrophorese, mittels Kapillarelektrophorese, mittels Sequenzierung und/oder mittels einer Hybridisierungstechnik auf einem DNA-Array, einem Bead-System oder einer anderen optischen, elektrischen oder elektrochemischen Messung erfolgt.
